Europäisches Patentamt

European Patent Office

Office européen des brevets

(11) Publication number: **0 073 888**
**B1**

# (12) EUROPEAN PATENT SPECIFICATION

(45) Date of publication of patent specification: **23.04.86**

(51) Int. Cl.⁴: **A 61 M 1/34, B 01 D 13/00**

(21) Application number: **82105520.9**

(22) Date of filing: **23.06.82**

(54) **Apparatus for removing heavy metal ions from blood.**

(30) Priority: **29.06.81 US 278631**

(43) Date of publication of application:
**16.03.83 Bulletin 83/11**

(45) Publication of the grant of the patent:
**23.04.86 Bulletin 86/17**

(84) Designated Contracting States:
**AT CH DE FR GB IT LI NL**

(56) References cited:
**DE-A-2 558 363**
**GB-A-1 528 475**
**US-A-4 013 564**

(73) Proprietor: **Ambrus, Clara, M.**
**143, Windsor Avenue**
**Buffalo, N.Y. 14209 (US)**
(73) Proprietor: **Horvath, Csaba**
**Pine Crest Road 69**
**Orange, Conn., 06472 (US)**

(72) Inventor: **Ambrus, Clara, M.**
**143, Windsor Avenue**
**Buffalo, N.Y. 14209 (US)**
Inventor: **Horvath, Csaba**
**Pine Crest Road 69**
**Orange, Conn., 06472 (US)**

(74) Representative: **Rasper, Joachim, Dr.**
**Bierstadter Höhe 22**
**D-6200 Wiesbaden (DE)**

Courier Press, Leamington Spa, England.

## Description

### Background of the Invention

This invention relates to an apparatus for removing toxic quantities of heavy metals from the bloodstream.

It is already known that substantial health problems are encountered by those who build up excessive amounts of heavy metal within their bodies. Particularly well known toxic metals which are often present in contaminated environments are iron, cadmium, lead, radioactive metals and mercury. However, physiological disorders can result in a patient building up toxic quantities of metals (iron-copper) in the bloodstream even when the environment is normal.

Many methods of treatment for heavy-metal poisoning are based on the use of specific agents which convert the metal to a chemical form in which it is relatively inert and can be readily excreted. Such treatments take time and are quite specific and consequently, depend excessively on a precise diagnosis. Moreover, they are more time consuming than is desirable.

GB—A—1 528 475 teaches a hollow fiber system which is not applicable to the removal of heavy metals from physiological fluids. It employs a low molecular weight chelator dissolved in an organic liquid substantially immiscible with water. In clinical applications trace amounts of organic liquids entering the blood can have toxic effects. Consequently a device described in this patent would never be approved for use in extracorporeal circulation systems.

DE—A—2 558 363 teaches a membrane system for removal of low molecular weight substances, such as metabolic products, from blood. The membrane has a swellable polymeric outer layer in admixture with an adsorbent, such as charcoal. The adsorbent can be also a synthetic resin having complex-building properties. This known system is, therefore, characterized by strong ultrafiltration flux through the membrane, and it requires the sucking action of a swellable polymer. Also, this reference does not teach the removal from blood of heavy metal ions which are to be bound by a chelating agent.

As described below, the present invention is intended to overcome some of these problems.

### Summary of the Invention

It is a principal object of the present invention to provide an improved apparatus for removal of heavy metal ions from blood.

Other objects of the invention will be obvious to those skilled in the art on reading this disclosure.

The above objects have been substantially achieved by construction and use of a device in which blood to be purified is passed over an ultrafiltration membrane which is in intimate contact on the non-blood-wetted side of the membrane with an immobilized chelating agent which forms means to accept and immobilize heavy metal ions, and which is dispersed in an aqueous medium.

The ultrafiltration membrane is preferably an anisotropic membrane with the tight or retention side facing the bloodstream. The membrane is conveniently formed of any number of polymers known to the art. The degree of blood compatibility of the membrane need not be high, because the apparatus will usually be discarded or cleaned after anything from a few minutes to a few hours of service. Nevertheless, some attention should be given to avoiding use of surfaces which are known to interact unfavourably with blood and have no particular benefit for the application being described.

One membrane that is suitable for use is a polysulfone membrane sold by Amicon Corporation under the trade designation P1O. It is the membrane that is used in Amicon's HIP1O ultrafiltration module. That membrane has a nominal molecular weight retention rating of 10,000.

The chelating agent can be an aqueous slurry or solution of the chelating resin or a relatively static mass. It is also possible to utilize liquid chelating macromolecules that cannot pass through the membrane but are in static or dynamic contact with the opposite side of the membrane from the physiological fluid being processed.

It has been found that, even though some quantity of a toxic metal atom will be carried on organic molecules which are large enough to be retained in the bloodstream, a significant number of metal atoms are with equilibrium in smaller compounds. As these smaller molecules or metal ions permeate the membrane the metal ion can be captured by the chelating agent, the equilibrium reestablishes itself to assure the availability of still additional metal ions to the chelate. This procedure continues until the concentration of metal in the blood is substantially reduced, i.e. reduced to a physiologically tolerable level. In many instances, it is possible to reduce the concentration of metal to acceptable levels within minutes. Occasionally, the limiting factor in the removal procedure will be the gradual removal from a patients tissue into the bloodstream. Treatment of several hours or for short periods spaced over a period of several days or weeks may be desirable in such a situation. Among the heavy metals which can be removed by a typical multivalent chelating agent are mercury, copper, nickel, cobalt, zinc, iron, cadmium, lead, uranium (UO + 2), manganese, berylium and plutonium.

Multi-valent chelating agents are preferred for use in the invention. The chelating resin which is used should be in a neutralized form. Chelating ion exchange resins are advantageously used. The sodium form of a material sold under the trade designation Chelex 100 by BIO-Rad Laboratories is useful when properly prepared and intimately contacted with the membrane as will be described below.

Among other chelating agents that can be immobilized in the porous outer structure of the membrane are aminoacetic acid derivatives such as (EDTA) and diethylene triaminopenta acetic acid (DTPA), hydroxamic acids of natural origin such as deferoxamine and rhodotorulic acid.

The use of an anisotropic membrane has a number of advantages. Undesirable contact interaction between the solid resin and the blood are minimized, and the desired ion transport is maximized with a minimum effect on the blood by the chelating resin.

It is preferable that the membrane be used in the tubular form in order to facilitate the maintenance of appropriate flow velocities, and flow distribution, of blood over the membrane surface. Velocities that are too high, even locally, can result in excessive damage to the blood. On the other hand, velocities that are too low tend to permit an accumulation of a gel-like barrier layer on the surface of the membrane and markedly reduce mass transport across the retentive membrane barrier layer. In general, velocities known to the blood processing art, e.g. those used in dialysis are suitable for use in the process of the invention.

Anisotropic membranes, i.e. those having a very thin barrier layer in contact with the blood and a more porous substructive in support are particularly useful in the process of the invention for they allow the chelating resin to be brought into intimate contact with the thin barrier membrane, impregnated well into the more grossly porous substructure of the membrane. Indeed, this is a particularly desirable configuration of the invention.

To facilitate this packing, the chelating resin is reduced to a paste or slurry which can be caused to pack closely around the exterior of closely packed membrane in parallel with one another.

Flow rates may vary widely, e.g. between about 0.03 ml per minute to about 3 ml per minute per ultrafilter tube. Rates between 1 and 2 ml per minute are preferred. These preferred rates indicate average linear blood flow of from about 20 to about 100 ml per minute through the Amicon P10 tubular ultrafilter.

Although, it is feasible to process whole blood using the apparatus of the invention, it is also possible to treat other physiological fluids. For example, blood fractions may be processed when, for some reason, this is appropriate for removal of a particular metal.

The nominal retention values of a membrane which are referred to herein are known in the art to be appropriately determined with dilute solutions of standard materials, for example a dextran polymer in 0.1% solution of the type sold under the trade designation "Blue Dextran".

Illustrative Example of the Invention

In this application and accompanying drawings there is shown and described a preferred embodiment of the invention and suggested various alternatives and modifications thereof, but it is to be understood that these are not intended to be exhaustive and that other changes and modifications can be made within the scope of the invention. These suggestions herein are selected and included for purposes of illustration in order that others skilled in the art will more fully understand the invention and the principles thereof and will be able to modify it and embody it in a variety of forms, each as may be best suited in the condition of a particular case.

In the Drawings

In the Drawings:

Fig. 1 is a partially schematic view of an apparatus useful in practice of the invention.

Fig. 2 is a section of Fig. 1.

Fig. 3 is a greatly enlarged cross-section of a hollow-fiber membrane structure constructed according to the invention.

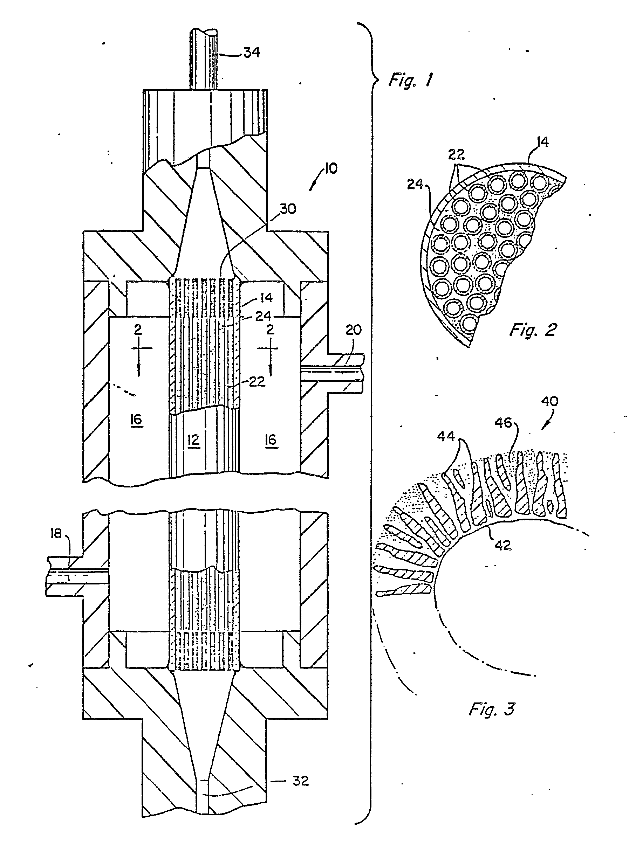

Referring to Figure 1, it is seen that a blood processing cartridge 10 is formed of an interior, blood-processing chamber 12 formed of interior glass wall 14. Around chamber 12 is an exterior chamber 16 which is utilized to control the temperature in chamber 12; a coolant the temperature of which is kept constant, is passed into chamber 16 through lower conduit 18 and passes out of the chamber through upper conduit 20.

Chamber 12 contains about five hundred tubular, anisotropic, ultrafiltration membranes 22 of the type generally called "hollow fibre membrane". In face, these membranes are about 0.3 millimeters in inside diameter and 0.5 millimeters in outside diameter. They have interior retentive membrane walls of about 1 micron in thickness and having a nominal central conduit path of about 8 inches.

The membranes are sealed together in matrices of resin 30 at the inlet port 32, outlet port 34. The resin effectively seals any cross-sectional area which may be left between the membranes and assures all blood entering the apparatus through inlet 32 flows into the tubular membranes 22.

Around membranes 22 is a mass of chelating resin 24. In operation about 100 ml per minute of blood is flowed through the 70 tubes for a period of 60 minutes. The iron content of the plasma is reduced from 50 mg percent to 3 mg percent in 3 passes. Most of the iron is removed from the plasma in about 3 minutes. Similar results are achieved with other heavy metals.

Preparation of the Chelating Resin

One suitable way of preparing the chelating resin for use is as follows:

The commercial resin, Chelax 100, was vacuum dried at 80°C for 24 hours. The dry particles of resin were ballmilled to obtain a fine powder having an average particle size of about 5 microns in diameter.

A quantity of 249 grams of the powder was washed with 500 ml of one normal HCl in which it was allowed to soak for 24 hours. The powder was then washed with 1000 ml distilled water, washed with 500 ml 1 normal sodium hydroxide in which it was allowed to soak for 24 hours before being given a final wash with 1000 ml of distilled water. These washings were such that, after each washing, the solid material was allowed to settle and the supernatant liquid was decanted. Before the settling step following the last wash, suspended solids were neutralized with 1 normal HCl.

The material remaining was a paste which, upon being dried at 100°C yielded 0.17 gram of dry Chelex 100 powder per gram of wet paste.

The cartridge is prepared by slurrying it in water and sucking the slurry into the shell through those spaces existing between the tubes mounted in place within the shell. It was found desirable to shake or vibrate the apparatus during the filling procedure. This aided the obtaining of intimate contact between the outer walls of the membranes and the slurry particles. Also, from time to time, water was added to help redistribute some of the resin particulate matter. After filling the residual moisture was removed by vacuum drying before the tubes were sealed into place.

A total of about 3 grams of chelating resin was left on the exterior of the membrane surfaces.

Referring to Figure 3, it is seen that a hollow fiber membrane structure 40 is composed of a single polymeric material which is formed into a tubular section comprising a relatively tight, very thin ultrafiltration membrane 42 and a relatively porous, exterior structure 44. In the pores 44 have been immobilized chelating agent 46 such as, e.g. a poly (dicarboxylmethyl-iminomethyl) styrene resin compound or macromolecular liquid chelating agent.

## Claims

1. An apparatus for removing heavy metal ions from a physiological fluid comprising a membrane ultrafilter, a flow path directing said fluid to contact the retentive side of said membrane and positioned in intimate contact with the opposite side of said membrane a medium containing a chelating agent, characterized in that said chelating agent is retained on said opposite side of said membrane, by being dispersed in an aqueous medium.

2. An apparatus as defined in claim 1 wherein said chelating agent is a finely-divided, multivalent resin composition.

3. An apparatus as defined in claim 2 wherein said resin composition is below 10 microns in average particle size and where said ultrafiltration membrane has a nominal molecular-weight retention value below about 50,000.

4. An apparatus as defined in claim 1, 2 or 3 wherein said retention value is about 10,000.

5. An apparatus as defined in claim 1, 2 or 3 wherein said chelating resin comprises multivalent carboxylate groups thereon.

6. An apparatus as defined in claim 1, 2 or 3 wherein said chelating agent is packed about the exterior of a plurality of small tubes which, taken together, form said ultrafiltration membrane.

## Patentansprüche

1. Vorrichtung zur Entfernung von Schwermetall-Ionen aus einer physiologischen Flüssigkeit, enthaltend ein Membran-Ultrafilter, wobei ein Strömungsweg diese Flüssigkeit derart ausrichtet, daß sie mit der retendierenden Seite dieser Membran in Berührung kommt, und wobei in innigem Kontakt mit der gegenüberliegenden Seite dieser Membran ein Medium angeordnet ist, das ein Chelat-bildendes Mittel enthält, dadurch gekennzeichnet, daß dieses Chelat-bildende Mittel auf der gegenüberliegenden Seite dieser Membran dadurch festgehalten wird, daß es in einem wässrigen Medium dispergiert ist.

2. Vorrichtung nach Anspruch 1, dadurch gekennzeichnet, daß dieses Chelat-bildende Mittel eine fein-zerteilte, multivalente Harzzusammensetzung ist.

3. Vorrichtung nach Anspruch 2, dadurch gekennzeichnet, daß diese Harzzusammensetzung eine durchschnittliche Teilchengröße von unter 10 Mikron aufweist und daß diese Ultrafiltrationsmembran einen Nenn-Molekulargewichtretentionswert von unter etwa 50000 hat.

4. Vorrichtung nach einem der Ansprüche 1, 2 oder 3, dadurch gekennzeichnet, daß dieser Retentionswert etwa 10000 beträgt.

5. Vorrichtung nach einem der Ansprüche 1, 2 oder 3, dadurch gekennzeichnet, daß dieses Chelat-bildende Harz mehrwertige Carboxylat-Gruppen trägt.

6. Vorrichtung nach einem der Ansprüche 1, 2 oder 3, dadurch gekennzeichnet, daß dieses Chelat-bildende Mittel um das Äußere einer Vielzahl von schmalen Röhren gepackt ist, die, zusammengenommen, diese Ultrafiltrationsmembran bilden.

## Revendications

1. Un appareil pour enlever des ions de métaux lourds d'un fluide physiologique, comprenant un ultrafiltre à membrane, un trajet d'écoulement dirigeant ledit fluide jusqu'au contact avec le côté de rétention de ladite membrane ainsi qu'un milieu contenant un agent de chélation et positionnée en contact intime avec le côté opposé de ladite membrane, caractérisé en ce que ledit agent de chélation est retenu sur ledit côté opposé de ladite membrane en étant dispersé dans un milieu aqueux.

2. Un appareil tel que défini dans la revendication 1, dans lequel ledit agent de chélation est une composition de résine multivalente et finement divisée.

3. Un appareil tel que défini dans la revendication 2, dans lequel ladite composition de résine a une dimension moyenne de particules inférieure à 10 microns et où ladite membrane d'ultrafiltration a une valeur nominale de rétention de poids moléculaire inférieure à environ 50 000.

4. Un appareil tel que défini dans la revendication 1, 2 ou 3, dans lequel ladite valeur de rétention est d'environ 10 000.

5. Un appareil tel que défini dans la revendication 1, 2 ou 3, dans lequel ladite résine de chélation comprend des groupes carboxylate multivalents.

6. Un appareil tel que défini dans la revendication 1, 2 ou 3, dans lequel ledit agent de chélation est compacté autour de l'extérieur d'une pluralité de petits tubes qui, maintenus ensemble, forment ladite membrane d'ultrafiltration.

Fig. 1

Fig. 2

Fig. 3

1